# EUROPÄISCHE PATENTSCHRIFT

(11) **EP 1 406 904 B1**
(45) Veröffentlichungstag und Bekanntmachung des Hinweises auf die Patenterteilung: **04.10.2006**
(21) Anmeldenummer: 02782456.4
(22) Anmeldetag: 04.07.2002
(51) Int. Cl.: C07D 487/08

(54) **VERFAHREN ZUR SELEKTIVEN SYNTHESE VON TRIETHYLENDIAMIN**
METHOD FOR SELECTIVELY SYNTHESISING TRIETHYLENEDIAMINE
PROCEDE DE SYNTHESE SELECTIVE DE TRIETHYLENEDIAMINE

(30) Priorität: 05.07.2001 DE 10132499
(43) Veröffentlichungstag der Anmeldung: 14.04.2004
(73) Patentinhaber: BASF Aktiengesellschaft, 67056 Ludwigshafen (DE)
(72) Erfinder: FRAUENKRON, Matthias, 67251 Freinsheim (DE); STEIN, Bernd, 64665 Alsbach-Hähnlein (DE); LANG, Ortmund, 66909 Quirnbach (DE)
(74) Vertreter: Isenbruck, Günter
(86) Internationale Anmeldenummer: PCT/EP2002/007430
(87) Internationale Veröffentlichungsnummer: WO 2003/004499

(56) Entgegenhaltungen:
- EP-A- 0 158 319
- EP-A- 0 312 734

## Beschreibung

Die vorliegende Erfindung betrifft ein selektives Verfahren zur Herstellung von Triethylendiamin (TEDA) aus Piperazin (PIP), bei dem ein Zeolith einer bestimmten Zusammensetzung als Katalysator eingesetzt wird.

TEDA (IUPAC-Name: 1,4-Diazabicyclo(2,2,2)-octan) ist ein wichtiges Zwischen- und Endprodukt in der chemischen Industrie, das hauptsächlich als solches bei der Polyurethanherstellung als Katalysator eingesetzt wird. Zur Herstellung von TEDA existiert eine große Anzahl verschiedener Synthesen, die sich hauptsächlich in der Wahl der Edukte und der benutzten Katalysatoren unterscheiden.

Zum einen ist der Einsatz einer Palette verschiedener Edukte möglich, die einen C2-Baustein und/oder einen Stickstoffbaustein enthalten und cyclisch oder acyclisch sein können. Beispiele geeigneter Edukte umfassen Ethylendiamin, Diethylentriamin, Ethanolamin, Aminoethylethanolamin, Piperazin, Aminoethylpiperazin und Hydroxyethylpiperazin. Es wird häufig ein einziges Edukt eingesetzt, auch Gemische von zwei oder mehreren geeigneten Edukten lassen sich jedoch vorteilhaft verwenden. Üblicherweise wird dem Reaktionsgemisch noch Wasser zugesetzt. Durch die Wahl der Edukte wird die Zusammensetzung des Produktgemischs entscheidend beeinflusst, wobei insbesondere die Vermeidung der Bildung von Nebenprodukten, neben der Verfügbarkeit der Ausgangsprodukte, ein wichtiger Aspekt im Hinblick auf die zu erreichende Spezifikation in der Aufarbeitung ist. In den meisten Fällen wird zur Erhöhung der Selektivität zu dem gewünschten Produkt TEDA die Synthese so durchgeführt, dass lediglich ein Teilumsatz des oder der eingesetzten Edukte eintritt. Der Nachteil der geringen Ausbeute wird wegen der erreichbaren niedrigen Mengen an unerwünschten Nebenprodukten in Kauf genommen.

Zum anderen können unterschiedliche Katalysatoren eingesetzt werden, beispielsweise nicht-kristalline Phosphate und Silikate verschiedener Herkunft, insbesondere jedoch Zeolithe. Auch bei den Zeolithen ist eine breite Variation hinsichtlich charakteristischer Parameter wie beispielsweise der Zusammensetzung, Vorbehandlung, Acidität oder Porengröße möglich. So können etwa, neben den klassischen, aus SiO₂ und Al₂O₃ bestehenden Zeolithen auch solche eingesetzt werden, die beispielsweise B, Ti, Ga oder andere Elementoxide enthalten. Auch bei den Zeolith-Katalysatoren richtet sich deren Einsatz prinzipiell nach den Selektivitäten, die mit diesen erzielt werden können.

Ein vorteilhaftes Edukt zur TEDA-Herstellung ist aufgrund seiner Bildung bei der Ethylendiamin-Synthese Piperazin (PIP), an das noch eine Ethylengruppe angelagert werden muß, um TEDA zu erhalten. Um diesen Baustein zur Verfügung zu stellen, wird PIP häufig im Gemisch mit einem weiteren Edukt, das den Baustein enthält, in der TEDA-Synthese umgesetzt. Dieses weitere Edukt kann beispielsweise Ethanolamin, Ethylendiamin oder Monoethylenglykol sein. Alternativ wird auch häufig ein substituiertes Piperazin eingesetzt, das in dem Substituenten den C2-Baustein enthält, etwa Aminoethylpiperazin oder Hydroxyethylpiperazin, wobei in einer Variante auch Gemische aus einem solchen substituierten Piperazin mit unsubstituiertem Piperazin zum Einsatz kommen.

Zum einen ist aber aus verfahrenstechnischen Gründen die Verwendung von Gemischen verschiedener Edukte benachteiligt, da die daraus erhältlichen Produktgemische komplexer zusammengesetzt sind als bei Einsatz von einzelnen Edukten und oft aufwendig aufgetrennt werden müssen. Zum anderen werden substituierte Piperazine generell aus unsubstituierten Piperazinen hergestellt, so dass es wünschenswert ist, auf Piperazin als einziges Edukt zurückgreifen zu können. Dies ist prinzipiell möglich und bekannt, wobei jedoch Umsatz und Selektivität prinzipiell bei niedrigeren Werten liegen als bei Einsatz etwa eines Gemischs verschiedener Edukte. Zur Lösung dieses Problems wird beispielsweise in der EP-A 312 734 ein Verfahren zur Herstellung von TEDA offenbart, bei dem als einziges Edukt PIP eingesetzt und der benutzte Zeolith-Katalysator die allgemeine Formel

(M^{I}•M^{II}O₂)•aM^{III}O₂•bH₂O

aufweist, in der
- M^{I}: ein Äquivalent eines einwertigen Alkalimetallions, ein Proton, eine Amoniumgruppe oder ein halbes Äquivalent eines zweiwertigen Erdalkalimetallkations ist,
- M^{II}: ein Äquivalent von Al³⁺, B³⁺, Sb³⁺, As³⁺, Cr³⁺, V³⁺, Ga³⁺ oder Fe³⁺,
- M^{III}: ein Äquivalent von Si⁴⁺, Ge⁴⁺, Ti⁴⁺ oder Zr⁴⁺ ist,
- a: einen Wert zwischen 15 und 200 und
- b: einen Wert zwischen 0 und 8 besitzt.

Der eingesetzte Katalysator weist dabei vorzugsweise eine Pentasil-Struktur auf, insbesondere eine ZSM-5-Struktur. Bezüglich der Zusammensetzung eines solchen Zeolithen ist es bevorzugt, wenn M^{I} ein Proton, M^{II} B³⁺ oder vorzugsweise Al³⁺, und M^{III} Ge⁴⁺ oder vorzugsweise Si⁴⁺ ist.

Das in der EP-A 312 734 offenbarte Verfahren erlaubt zwar hohe Selektivitäten zu TEDA von bis zu ca. 90 %, wie aus den Beispielen hervorgeht. Jedoch entsteht immer eine bestimmte Menge von 2-Ethylpiperazin (ETPIP) als Nebenprodukt, das äußerst schwierig von TEDA abzutrennen ist. Durch Anlegen niedriger Reaktionstemperaturen läßt sich die Bildung von EtPIP zwar auf sehr niedrige Werte reduzieren, dies wird jedoch durch einen sehr niedrigen Umsatz erkauft. So ist es nach Beispiel 3 zwar möglich, das Verfahren so durchzuführen, dass nur 0,21 % EtPIP (entsprechend einer Selektivität von 0,21 %) gebildet werden. Solche Selektivitäten liegen in einem Bereich, der für ein Verfahren im industriellen Maßstab akzeptabel ist. Dabei beträgt der Umsatz von PIP jedoch nur 18 %, was wiederum deutlich zu niedrig ist.

Die Aufgabe der vorliegenden Erfindung besteht darin, ein Verfahren zur Verfügung zu stellen, mit dem die Produktion von TEDA aus PIP mit hohen Umsätzen und Selektivitäten zu TEDA und nur geringer Bildung von EtPIP erreicht werden kann.

Diese Aufgabe wird gelöst durch ein Verfahren zur Herstellung von TEDA durch Umsetzung von PIP an einem Zeolithen, der neben SiO₂ mindestens ein weiteres Oxid mindestens eines zwei-, drei- oder vierwertigen Metalls M enthält, wobei das Verfahren dadurch gekennzeichnet ist, dass der Zeolith ein molares Verhältnis Si/M von > 100 aufweist.

Es wurde gefunden, dass durch den Einsatz der vorstehend beschriebenen Zeolithe die Bildung von EtPIP bei der Synthese von TEDA aus PIP unterdrückt wird. Der Einsatz des Zeolithen gestattet das Durchführen des Verfahrens bei Temperaturen, die über den gemäß EP-A 312 734 verwendeten liegen, wodurch der PIP-Umsatz steigt, ohne dass unerwünschte hohe Mengen an EtPIP gebildet werden.

Die erfindungsgemäß eingesetzten Zeolithe enthalten in ihrem Gerüst neben SiO₂ ein oder mehrere Oxide eines Metalls M in den Oxidationsstufen II, III oder IV, also Oxide der Zusammensetzung M^{II}O, M^{III}₂O₃ und/oder M^{IV}O₂. Ein wichtiges Charakteristikum der erfindungsgemäß eingesetzten Zeolithen ist das molare Verhältnis Si zu dem Metall M, das sogenannte Modul, das bei Werten von > 100, vorzugsweise > 200, mehr bevorzugt > 300 bis 40000, insbesondere 400 bis 5000 liegt.

Die obere Grenze des Moduls (40000) ist nur durch die Reinheit der Ausgangsubstanzen (Restspuren an M bzw. Verbindungen von M) und die Reinheit und chemische Beständigkeit der bei der Synthese des Zeoliths eingesetzten Apparate begrenzt.

Bei einem Modul unterhalb der angegebenen Grenze nimmt die Brönsted- und Lewis-Aciditätsdichte (Aciditätsdichte: saure Zentren/Katalysatorgesamtoberfläche) der Zeolithe deutlich zu, die erreichbare TEDA-Ausbeute und -Selektivität und die Katalysatorstandzeit deutlich ab und der Aufwand zur Aufreinigung des TEDAs deutlich zu.

Überraschenderweise wurde gefunden, dass durch die drastische Reduzierung der Acidität innerhalb des Zeolithkristalls im erfindungsgemäßen Verfahren, die durch den Einbau von 2- und/oder 3-wertigen Metallen in Form von Metalloxiden im Gitter im Normalfall während der hydrothermalen Synthese erzeugt wird, die erfindungsgemäßen

Vorteile erzielt werden, z.B. eine deutliche Verbesserung in der Selektivität bzgl. TEDA. Auch Zeolithe mit vierwertigen Metallen in Form von Metalloxiden im Zeolithgitter erzielen erfindungsgemäß eine verbesserte Selektivität bezüglich TEDA.

Für den Zeolith-Katalysator, bevorzugt vom Pentasil-Typ, mit Modulen wie oben angegeben bestehen weder zusätzliche Erfordernisse bezüglich des Zeolith-Materials als solchem noch bezüglich des Verfahrens nach dem dieses erhältlich ist.

Bei dem im erfindungsgemäßen Verfahren verwendeten Zeolith-Katalysator, der neben SiO₂ ein oder mehrere Metalle M in den Oxidationsstufen II, III oder IV als Oxide enthält, ist das Metall M in der Oxidationsstufe II bevorzugt ausgewählt aus der Gruppe Zn, Sn und Be oder Mischungen hiervon, das Metall M in der Oxidationsstufe III bevorzugt ausgewählt aus der Gruppe Al, B, Fe, Co, Ni, V, Mo, Mn, As, Sb, Bi, La, Ga, In, Y, Sc und Cr oder Mischungen hiervon und das Metall M in der Oxidationsstufe IV bevorzugt ausgewählt aus der Gruppe Ti, Zr, Ge, Hf und Sn oder Mischungen hiervon.

Bevorzugt sind Zeolithe, bei denen M für Aluminium, Gallium, Titan, Zirkonium, Germanium, Eisen oder Bor steht. Solche, bei denen M für Aluminium, Titan, Eisen und Bor steht, sind besonders bevorzugt.

Als erfindungsgemäß einzusetzende Zeolith-Katalysatoren des Pentasil-Typs sind z.B. folgende Typen geeignet: ZSM-5 (wie z.B. in US-A-3,702,886 offenbart), ZSM-11 (wie z.B. in US-A-3,709,979 offenbart), ZSM-23, ZSM-53, NU-87, ZSM-35, ZSM-48 und Mischstrukturen aus mindestens zwei der oben genannten Zeolithe, insbesondere ZSM-5 und ZSM-11, sowie deren Mischstrukturen.

Besonders bevorzugt sind für das erfindungsgemäße Verfahren Zeolithe mit MFI-, MEL-Struktur, MEL/MFI- oder MFI/MEL-Mischstruktur.

Die erfindungsgemäß verwendeten Zeolithe sind kristalline Metallsilikate mit geordneter Kanal- und Käfigstruktur, die Mikroporen aufweisen. Das Netzwerk solcher Zeolithe ist aufgebaut aus SiO₄ - und M_{2/z}O (z = 2, 3 oder 4) ― Tetraedern, die über gemeinsame Sauerstoffbrücken verbunden sind. Eine Übersicht über die bekannten Strukturen findet sich beispielsweise bei W.M. Meier, D.H. Olsen und Ch. Baerlocher in "Atlas of Zeolite Structure Types", Elsevier, 4. Auflage, London 1996.

Ferner sind erfindungsgemäß Zeolithe einsetzbar, die kein Aluminium (M = Al) enthalten und bei denen im Zeolithgitter das Si(IV) teilweise durch ein Metall M(IV), wie z.B. Ti, Zr, Ge, Hf und/oder Sn, teilweise durch ein Metall M(II), wie z.B. Zn, Sn und/oder Be und/oder teilweise durch ein Metall M(III), wie z.B. B, Fe, Co, Ni, V, Mo, Mn, As, Sb, Bi, La, Ga, In, Y, Sc und/oder Cr, ersetzt ist.

### (II = Oxidationsstufe 2, III = Oxidationsstufe 3, IV = Oxidationsstufe 4).

Überlicherweise stellt man die genannten Zeolithe dadurch her, dass man eine Mischung aus einer SiO₂-Quelle sowie aus einer Metall-Quelle (z.B. M = Al, Zn, Be, B, Fe, Co, Ni, V, Mo, Mn, As, Sb, Bi, La, Ga, In, Y, Sc, Cr, Ti, Zr, Ge, Hf und/oder Sn in den Oxidationsstufen wie oben beschrieben) und einer stickstoffhaltigen Base als Templat ("Schablonen-Verbindung"), wie z.B. Tetraalkylammoniumsalz, gegebenenfalls noch unter Hinzufügen basischer Verbindungen (z.B. Laugen), in einem Druckbehälter unter erhöhter Temperatur im Zeitraum mehrerer Stunden oder einiger Tage umsetzt, wobei ein kristallines Produkt entsteht. Dieses wird abgetrennt (z.B. abfiltriert, sprühgetrocknet oder ausgefällt), gewaschen, getrocknet und zur Entfernung der organischen Stickstoffbase bei erhöhter Temperatur kalziniert (siehe unten). Wahlweise ist auch die Synthese ohne Templat möglich, sofern die Bildung des Zeolithen gewährleistet ist. In dem so erhaltenen Pulver liegt das Metall (z.B. M = Al, Zn, Be, B, Fe, Co, Ni, V, Mo, Mn, As, Sb, Bi, La, Ga, In, Y, Sc, Cr, Ti, Zr, Ge, Hf und/oder Sn in den Oxidationsstufen wie oben beschrieben) zumindest teilweise innerhalb des Zeolithgitters in wechselndem Anteil mit 4-, 5- oder 6-facher Koordination vor.

Die erfindungsgemäß verwendeten Zeolithe sind über das beschriebene Verfahren herstellbar und/oder kommerziell im Handel erhältlich.

Liegt der erfindungsgemäß einzusetzende Zeolith-Katalysator, bevorzugt des Pentasil-Typs, aufgrund der Art der Produktion nicht zumindest teilweise in der bevorzugten aciden H⁺-Form und/oder NH₄⁺-Form vor, sondern z.B. in der Na⁺-Form (oder einer anderen beliebigen Metallsalzform), so kann dieser, gemäß dem Stand der Technik, durch lonenaustausch, z.B. mit Ammoniumionen, und anschließender Kalzinierung (s. unten) zumindest partiell in die bevorzugte H⁺- und/oder NH₄⁺-Form überführt werden. Die ebenso literaturbekannte, Behandlung mit verdünnter Protonen-Säure, z.B. Mineralsäure, zur Überführung des Zeolithen zumindest teilweise in die H⁺-Form ist genauso praktikabel. Geeignet sind hier alle Protonen-Säuren, wie z.B. Salzsäure oder Schwefelsäure (s. unten).

Anschließend ist es möglich, den so ausgetauschten Zeolith-Katalysator durch Ionenaustausch mit einer entsprechenden Metallsalzlösung (Metall Me = Alkalimetall, Erdalkalimetall, Übergangsmetall) in eine gewünschte Me⁺-Form zu überführen, die noch H⁺ und/oder NH₄⁺ enthält.

Um eine möglichst hohe Selektivität, hohe Umsätze sowie besonders lange KatalysatorStandzeiten zu erreichen, kann es vorteilhaft sein, die anspruchsgemäßen Zeolith-Katalysatoren zu modifizieren.

Eine geeignete Modifizierung der Zeolith-Katalysatoren besteht darin, wie in ,J. Weitkamp et al., Catalysis and Zeolites, Kap. 3: Modification of Zeolites, Springer Verlag, 1999' beschrieben, dass man das zeolithische Material - verformt oder unverformt - einer Behandlung, gemäß bekanntem Stand der Technik (EP-A-382 055, S. 4, Zeile 2ff + Zeile 20ff; DE-C2-24 34 913, S. 3 Zeile 23ff; US-A-5,041,548, S. 4, Zeile 27ff), mit konzentrierten oder verdünnten Protonen-Säuren - wie z.B. Salzsäure, Schwefelsäure, Flusssäure, Phosphorsäure, einer Carbonsäure, Dicarbonsäure oder Polycarbonsäure - und/oder Komplexbildnern - wie z.B. Acetylacetonat (acac), Nitrilotriessigsäure,

Sulfosalicylsäure, Ethylendiaminotetraessigsäure (EDTA) -, z.B. gemäß EP-A-842 936 und RU-C1-21 14 849, und/oder Wasserdampf unterwirft.

In einer besonderen Ausführungsform kann eine Dotierung der im erfindungsgemäßen Verfahren verwendeten Zeolithe durch Auftragen von Übergangsmetallen der I. bis VIII. Nebengruppe, bevorzugt die der I., II., IV. und VIII. Nebengruppe, besonders bevorzugt Zn, Ti, Zr, Fe, Co, Ni, Cr, V, auf diese erfolgen.

Die Auftragung kann durch Tränken des im erfindungsgemäßen Verfahren verwendeten Zeoliths in wässrigen Metallsalzlösungen, durch Aufsprühen entsprechender Metallsalzlösungen auf den Zeolith oder durch andere geeignete, im Stand der Technik bekannte Verfahren erreicht werden. Als Metallsalze zur Herstellung der Metallsalzlösungen eignen sich die Nitrate, Nitrosylnitrate, Halogenide, Carbonate, Carboxylate, Acetylacetonate, Chlorokomplexe, Nitrokomplexe oder Aminkomplexe der entsprechenden Metalle, wobei die Nitrate und Nitrosylnitrate bevorzugt sind. Bei Zeolithen, die mit mehreren Metallen dotiert sind, können die Metallsalze bzw. Metallsalzlösungen gleichzeitig oder nacheinander aufgebracht werden.

Die mit den Metallsalzlösungen beschichteten oder getränkten Zeolithe werden anschließend, vorzugsweise bei Temperaturen zwischen 60 und 150°C, getrocknet und wahlweise bei Temperaturen zwischen 200 und 950°C, vorzugsweise zwischen 400 und 750°C, kalziniert. Bei getrennter Auftränkung wird der Katalysator nach jedem Tränkschritt getrocknet und wahlweise, wie oben beschrieben, kalziniert. Die Reihenfolge in der die Übergangsmetalle aufgetränkt werden, ist dabei frei wählbar. Wahlweise werden anschließend die beschichteten und getrockneten sowie wahlweise kalzinierten Zeolithe durch Behandlung in einem Gasstrom, der freien Wasserstoff enthält, bei Temperaturen zwischen 30 und ungefähr 600°C, vorzugsweise zwischen 150 und ungefähr 450°C, aktiviert. Vorzugsweise besteht der Gasstrom aus 50 bis 100 Vol.-% Wasserstoff und 0 bis 50 Vol.-% Stickstoff.

Die Übergangsmetalllösungen werden in einer solchen Menge auf den Zeolith aufgebracht, dass der Gesamtgehalt an Übergangsmetall, jeweils bezogen auf das Gesamtgewicht des Katalysators, ungefähr 0,01 bis ungefähr 10 Gew.-%, vorzugsweise ungefähr 0,01 bis 5 Gew.-%, weiter bevorzugt ungefähr 0,01 bis ungefähr 2 Gew.-% und inbesondere ungefähr 0,05 bis 1 Gew.-% beträgt.

Die Übergangsmetalloberfläche auf dem Katalysator beträgt dabei insgesamt vorzugsweise ungefähr 0,01 bis ungefähr 10 m²/g, weiter bevorzugt 0,05 bis 5 m²/g und inbesondere ungefähr 0,05 bis 3 m²/g (m² pro g des Katalysators). Die Metalloberfläche wird mittels der von J. LeMaitre et al. in "Characterization of Heterogeneous Catalysts", Hrsg. Francis Delanny, Marcel Dekker, New York 1984, S. 310 - 324, beschriebenen Chemisorptionsverfahren bestimmt.

Zur Erhöhung der Standfestigkeit können die erfindungsgemäß einzusetzenden Zeolithe geträgert werden, z.B. auf Cellulosematerialien, Tonen, Polymere, Metalle, Graphit, Bindemitteln oder Metalloxiden wie Tonerden, Aluminiumoxid, Siliciumdioxid. Weiterhin ist es möglich diese als Granulat, in Kugelform oder auf Glas- oder andere Körper wie z.B. Geweben (insbesondere Metallgeweben) jeglicher Art aufgebracht einzusetzen.

Als verfestigende Formgebungsprozesse für die erfindungsgemäß einzusetzenden Zeolithe können im Prinzip alle Methoden zur Erlangung einer entsprechenden Formung verwendet werden. Bevorzugt werden Verfahren, bei denen die Formgebung durch Tablettierung oder Extrusion erfolgt. Besonders bevorzugt werden Verfahren, bei denen die Formgebung durch Extrusion in üblichen Extrudern, beispielsweise zu Strängen mit einem Durchmesser von üblicherweise 1 bis 10 mm, insbesondere 2 bis 5 mm, erfolgt. Werden Bindemittel und/oder Hilfsmittel benötigt, ist der Extrusion bzw. der Tablettierung zweckmäßigerweise ein Mischungs- oder Knetprozess vorgeschaltet. Gegebenenfalls erfolgt nach der Extrusion/Tablettierung noch ein Kalzinierungsschritt. Die erhaltenen Formkörper werden gewünschtensfalls zerkleinert, vorzugsweise zu Granulat oder Splitt mit einem Partikeldurchmesser von 0,5 bis 5 mm, inbesondere 0,5 bis 2 mm. Dieses Granulat oder dieser Splitt und auch auf anderem Wege erzeugte Katalysatorformkörper enthalten praktisch keine feinkörnigeren Anteile als solche mit 0,5 mm Mindestpartikeldurchmesser.

In einer bevorzugten Ausführungsform enthält der geformte, erfindungsgemäß einzusetzende Zeolith bis zu 80 Gew.-% Bindemittel, bezogen auf die Gesamtmasse des Katalysators. Besonders bevorzugte Bindemittelgehalte sind 1 bis 60 Gew.-%, insbesondere 20 bis 45 Gew.-%. Als Bindemittel eignen sich im Prinzip alle für derartige Zwecke eingesetzte Verbindungen, bevorzugt werden Verbindungen, inbesondere Oxide, des Siliciums, Aluminiums, Bors, Phosphors, Zirkoniums und/oder Titans. Von besonderem Interesse als Bindemittel ist Siliciumdioxid, wobei das SiO₂ auch als Kieselsol oder in Form von Tetraalkoxysilanen in den Formgebungsprozess eingebracht werden kann. Auch als Bindemittel verwendbar sind Oxide des Magnesiums und Berylliums sowie Tone, z.B. Montmorillonit, Kaoline, Bentonite, Halloysite, Dickite, Nacrite und Anauxite.

Als Hilfsmittel für die verfestigenden Formgebungsprozesse sind beispielsweise Verstrangungshilfsmittel für die Extrusion zu nennen, ein übliches Verstrangungsmittel ist Methylcellulose. Derartige Mittel werden in der Regel in einem nachfolgendem Kalzinierungsschritt vollständig verbrannt.

Die Kalzinierung des erfindungsgemäß einzusetzenden Zeolith-Katalysators erfolgt bei Temperaturen von 250 bis 950°C, bevorzugt bei 400 bis 750°C, besonders bevorzugt bei 450 bis 600°C, für die Dauer von im allgemeinen mindestens einer Stunde, bevorzugt für 2 - 5 Stunden. Die Kalzinierung erfolgt in einer Gasatmosphäre, z.B. Stickstoff-, Luft-, Edelgas-Atmospäre. In der Regel wird in sauerstoffhaltiger Atmosphäre kalziniert, wobei der Sauerstoffgehalt 0,1 bis 90 Vol.-%, bevorzugt 0,2 bis 22 Vol.-%, besonders bevorzugt 10 bis 22 Vol.-%, beträgt. Die Verwendung von anderen Sauerstoff-liefernden Substanzen ist ebenfalls möglich. Der obige Begriff "Sauerstoff-liefernde Substanzen" umfasst alle Substanzen, die in der Lage sind, unter den angegebenen Kalzinierbedingungen Sauerstoff abzugeben. Insbesondere zu nennen sind: Stickoxide der Formel NₓO_{y}, wobei x und y so gewählt werden, dass sich ein neutrales Stickoxid ergibt, N₂O, N₂O haltiger Abgasstrom aus einer Adipinsäureanlage, NO, NO₂ Ozon oder ein Gemisch aus zwei oder mehr davon. Bei Verwendung von CO₂ als Sauerstoff-liefernde Substanz werden bevorzugt Temperaturen von 500°C bis 800°C während der Kalzination eingestellt. Eine Kalzinierung unter Wasserdampfatmosphäre ist ebenfalls möglich.

Erfindungsgemäß wurde weiterhin erkannt, dass nach dem Einsatz des erfindungsgemäß verwendeten Zeolith-Katalysators, dieser unabhängig von seiner Form, z.B. nach Abnahme der Aktivität und/oder der Selektivität, durch ein Verfahren regeneriert werden kann, bei dem die Regenerierung durch gezieltes Abbrennen der für die Deaktivierung verantwortlichen Beläge erfolgt. Dabei wird bevorzugt in einer Inertgasatmosphäre gearbeitet, die genau definierte Mengen an Sauerstoff-liefernden Substanzen enthält. Ein solches Regenerierungsverfahren ist unter anderem in der WO 98/55228 und der DE-A1-19 72 39 49 beschrieben, deren Offenbarung durch diesbezügliche Bezugnahme hiermit vollumfänglich auch zum Gegenstand der vorliegenden Anmeldung gemacht wird.

Nach der Regeneration ist die Aktivität und/oder die Selektivität des Katalysators, verglichen mit dem Zustand unmittelbar vor der Regeneration, erhöht.

Der zu regenerierende, erfindungsgemäß einzusetzende Zeolith-Katalysator wird entweder in der Umsetzungsvorrichtung (Reaktor) oder in einem externen Ofen in einer Atmosphäre, die 0,1 bis ungefähr 20 Volumen-Anteile von Sauerstoff-liefernden Substanzen, besonders bevorzugt 0,1 bis ungefähr 20 Volumen-Anteile Sauerstoff, enthält, auf eine Temperatur im Bereich von ungefähr 250°C bis 800°C, vorzugsweise ungefähr 400°C bis 550°C und insbesondere ungefähr 450°C bis 500°C, aufgeheizt. Dabei wird das Aufheizen vorzugsweise mit einer Aufheizrate von ungefähr 0,1 °C/Min. bis ungefähr 20°C/Min., vorzugsweise ungefähr 0,3°C/Min bis ungefähr 15°C/Min. und insbesondere 0,5°C/Min. bis 10°C/Min., durchgeführt.

Während dieser Aufheizphase wird der Katalysator bis zu einer Temperatur aufgeheizt, bei der die sich dort befindlichen, meist organischen Beläge zu zersetzen beginnen, während gleichzeitig die Temperatur über den Sauerstoffgehalt geregelt wird und somit nicht derart ansteigt, dass es zu Schädigungen der Katalysatorstruktur kommt. Das langsame Erhöhen der Temperatur bzw. das Verweilen bei niedriger Temperatur durch Einstellen des entsprechenden Sauerstoffgehaltes und der entsprechenden Heizleistung ist bei hohen organischen Beladungen des zu regenerierenden Katalysators ein wesentlicher Schritt zur Verhinderung einer lokalen Überhitzung des Katalysators.

Sinkt die Temperatur des Abgasstroms am Reaktorausgang trotz steigender Mengen an Sauerstoff-liefernden Substanzen im Gasstrom, so ist das Abbrennen der organischen Beläge beendet. Die Dauer der Behandlung beträgt im allgemeinen jeweils ungefähr 1 bis 30, vorzugsweise ungefähr 2 bis ungefähr 20 und insbesondere ungefähr 3 bis ungefähr 10 Stunden.

Beim anschließenden Abkühlen des so regenerierten Katalysators ist darauf zu achten, dass das Abkühlen nicht zu schnell erfolgt ("Abschrecken"), da sonst die mechanische Festigkeit des Katalysators negativ beeinflusst werden kann.

Es kann erforderlich sein, den Katalysator nach der durchgeführten Regeneration durch Kalzination, wie oben beschrieben, einer Spülung mit Wasser und/oder verdünnten Säuren, wie z.B. Salzsäure, zu unterziehen, um evtl. die durch Verunreinigung der Edukte verbleibende anorganische Beladung des Katalysators (Alkalispuren etc.) zu entfernen. Anschließend kann eine erneute Trocknung und/oder Kalzination des Katalysators durchgeführt werden.

In einer weiteren Ausführungsform des erfindungsgemäßen Verfahrens wird der zumindest teilweise deaktivierte Katalysator vor dem Aufheizen gemäß der Regenerationsprozedur mit einem Lösungsmittel im Umsetzungsreaktor oder in einem externen Reaktor gewaschen, um noch anhaftendes Wertprodukt zu entfernen. Dabei wird das Waschen so durchgeführt, dass zwar die jeweils am Katalysator anhaftenden Wertprodukte von diesem entfernt werden können, aber Temperatur und Druck nicht so hoch gewählt werden, dass die meist organischen Beläge ebenfalls entfernt werden. Vorzugsweise wird der Katalysator dabei mit einem geeigneten Lösungsmittel lediglich gespült. Somit eignen sich für diesen Waschvorgang alle Lösungsmittel, in denen sich das jeweilige Umsetzungsprodukt gut löst. Die benutzte Menge an Lösungsmittel sowie die Dauer des Waschvorgangs sind nicht kritisch. Der Waschvorgang kann mehrmals wiederholt und bei erhöhter Temperatur durchgeführt werden. Bei Verwendung von CO₂ als Lösungsmittel ist überkritischer Druck bevorzugt, ansonsten kann der Waschvorgang unter Normaldruck bzw. erhöhtem oder überkritischem Druck erfolgen. Nach der Beendigung des Waschvorgangs wird der Katalysator im allgemeinen getrocknet. Obwohl der Trocknungsvorgang im allgemeinen unkritisch ist, sollte die Trocknungstemperatur die Siedetemperatur des zum Waschen verwendeten Lösungsmittels nicht zu stark übersteigen, um ein schlagartiges Verdampfen des Lösungsmittels in den Poren, insbesondere in den Mikroporen zu vermeiden, da auch dies zu Schädigungen des Katalysators führen kann.

Eine bevorzugte Ausführung des Herstellverfahrens kann darin bestehen, dass das erfindungsgemäße, kontinuierliche Verfahren zur Synthese von TEDA bei der Regeneration des erfindungsgemäßen Katalysators nicht unterbrochen werden muss, um den Verfahrensdurchsatz zu steigern. Dies kann durch die Verwendung von mindestens zwei parallel verschalteten Reaktoren erreicht werden, die wechselweise betrieben werden können.

Die Katalysatorregeneration kann derart durchgeführt werden, dass mindestens einer der parallel geschalteten Reaktoren aus der jeweiligen Reaktionsstufe abgekoppelt wird und der in diesem Reaktor enthaltene Katalysator regeneriert wird, wobei im Laufe des kontinuierlichen Verfahrens in jeder Stufe immer mindestens ein Reaktor zur Umsetzung von PIP zur Verfügung steht.

Das erfindungsgemäß erhaltene TEDA kann zur Verbesserung seiner Reinheit aus geeigneten Lösungsmitteln (z.B. Pentan, Hexan) umkristallisiert werden. Meist ist dies jedoch nicht erforderlich, da TEDA nach dem erfindungsgemäßen Verfahren mit Reinheiten größer 95 Gew.-%, z.B. größer 97 Gew.-%, hergestellt werden kann.

In einer besonderen Ausgestaltung ist das anspruchsgemäße TEDA-Herstellverfahren kombiniert mit dem sich anschließenden TEDA-Verfahren gemäß der älteren EP-Anmeldung Nr. 00114475.7 vom 06.07.00 (BASF AG).

Gemäß dieser Kombination wird zunächst TEDA anspruchsgemäß hergestellt. Bei der sich anschließenden Aufarbeitung des TEDAs (z.B. destillativ), die mehrstufig sein kann, wird das TEDA, bevorzugt in der letzten Aufarbeitungsstufe (insbesondere Destillations- bzw. Rektifikationsstufe), verdampft und das, z.B. am Kopf oder in einem Seitenabzug der Destillationskolonne erhaltene, dampfförmige TEDA, das bevorzugt eine Reinheit von größer 95 Gew.-%, insbesondere von größer 97 Gew.-% besitzt, in ein flüssiges Lösungsmittel einleitet. Diese Einleitung des dampfförmigen TEDAs direkt in ein flüssiges Lösungsmittel wird im Folgenden auch, TEDA-Quench' genannt.

Durch anschließende Auskristallisation des TEDAs aus der so erhaltenen Lösung wird reines TEDA mit hoher Qualität erhalten.

Das flüssige Lösungsmittel wird im allgemeinen aus der Gruppe cyclische oder acyclische Kohlenwasserstoffe, chlorierte aliphatische Kohlenwasserstoffe, aromatische Kohlenwasserstoffe, Alkohole, Ketone, aliphatische Carbonsäureester, aliphatische Nitrile und Ether ausgewählt.

Zur Herstellung einer Lösung von reinem TEDA gemäß obiger Verfahrenskombination, die z.B. als Katalysatorlösung bei der Polyurethanschaumherstellung verwendet werden kann, wird als Lösungsmittel für den TEDA-Quench bevorzugt ein Alkohol (z.B. Ethylenglykol, 1,4-Butandiol, bevorzugt Dipropylenglykol) eingesetzt. Die Farbzahl einer so erhaltenen 33 Gew.-%igen TEDA-Lösung in Dipropylenglykol beträgt kleiner 150 APHA, insbesondere kleiner 100 APHA, ganz besonders kleiner 50 APHA.

Zur Herstellung von reinem (kristallinem) TEDA gemäß obiger Verfahrenskombination wird als Lösungsmittel für den TEDA-Quench bevorzugt ein aliphatischer Kohlenwasserstoff, insbesondere ein gesättigter aliphatischer Kohlenwasserstoff mit 5 bis 8 C-Atomen (wie z.B. Hexan, Heptan, bevorzugt Pentan) verwendet. Die Kristallisation des reinen TEDAs aus der erfindungsgemäß hergestellten TEDA-Lösung kann nach den dem Fachmann bekannten Verfahren erfolgen. Die durch eine nachfolgende mehrstufige, oder bevorzugt einstufige, Kristallisation erhaltenen TEDA-Kristalle sind hochrein (Reinheit von im allgemeinen mindestens 99,5 Gew.-%, insbesondere mindestens 99,8 Gew.-%. Gehalt an PIP kleiner 0,1 Gew.-%, insbesondere kleiner 0,05 Gew.-%, Gehalt an N-Ethylpiperazin kleiner 0,02 Gew.-%, insbesondere kleiner 0,01 Gew.-%) und die Farbzahl einer 33 Gew.-%igen Lösung in Dipropylenglykol beträgt kleiner 50 APHA, insbesondere kleiner 30 APHA.

### (Alle APHA-Zahlen nach DIN ISO 6271).

Die Einleitung des dampfförmigen TEDAs in das flüssige Lösungsmittel erfolgt in einem Quenchapparat, z.B. bevorzugt in einem Fallfilmkondensator (Dünnschicht-, Rieselfilm-oder Fallstromkondensator) oder in einem Düsenapparat. Dabei kann das dampfförmige TEDA im Gleich- oder im Gegenstrom mit dem flüssigen Lösungsmittel geführt werden. Vorteilhaft ist die Einleitung des dampfförmigen TEDAs von oben in den Quenchapparat. Weiterhin vorteilhaft ist die tangentiale Zufuhr des flüssigen Lösungsmittels am Kopf des Fallfilmkondensators oder die Zufuhr des flüssigen Lösungsmittels durch eine oder mehrere Düsen um eine vollständige Benetzung der Innenwand des Quenchapparates zu erreichen.

Im allgemeinen wird die Temperatur im TEDA-Quench durch Temperierung des eingesetzten Lösungsmittels und/oder des Quenchapparates auf 20 bis 100°C, bevorzugt 30 bis 60°C, eingestellt. Der Absolutdruck im TEDA-Quench beträgt im allgemeinen 0,5 bis 1,5 bar.

Im allgemeinen wird so verfahren, dass, je nach Art des Lösungsmittels, beim TEDA-Quench zunächst Lösungen mit einem TEDA-Gehalt von ca. 1 bis 50 Gew.-%, bevorzugt 20 bis 40 Gew.-%, erhalten werden.

Das erfindungsgemäß Verfahren wird bei einer Reaktionstemperatur von 250 bis 500°C, vorzugsweise 300 bis 400°C, insbesondere 330 bis 400°C durchgeführt. Die angewandten Drücke liegen bei 0,01 bis 50 bar, vorzugsweise 0,5 bis 20 bar, insbesondere bei Atmosphärendruck. In diese Werte ist der Druckverlust, der über das Katalysatorbett entsteht, nicht eingerechnet. PIP wird dabei generell im Gemisch mit Wasser eingesetzt, wobei vorzugsweise mindestens 10 Gew.-% Wasser, insbesondere 20 bis 60 Gew.-% Wasser, in dem Gemisch vorhanden sind.

Das erfindungsgemäße Verfahren kann diskontinuierlich oder bevorzugt kontinuierlich durchgeführt werden.

Die erfindungsgemäße Umsetzung kann in der Flüssigphase oder bevorzugt in der Gasphase durchgeführt werden.

Bevorzugt wird die Umsetzung in Gegenwart eines Lösungs- oder Verdünnungsmittels durchgeführt.

Als Lösungs- oder Verdünnungsmittel eignen sich zB. acyclische oder cyclische Ether mit 2 bis 12 Kohlenstoffatomen, wie Dimethylether, Diethylether, Di-n-Propylether oder dessen Isomere, MTBE, THF, Pyran, oder Lactone, wie gamma-Butyrolacton, Polyether, wie Monoglyme, Diglyme etc., aromatische oder aliphatische Kohlenwasserstoffe, wie Benzol, Toluol, Xylol, Pentan, Cyclopentan, Hexan und Petrolether, oder deren Gemische und besonders auch N-Methylpyrrolidon (NMP) oder Wasser oder wässrige organische Lösungs- oder Verdünnungsmittel der oben genannten Art. Weiterhin ist Ammoniak als Lösungs- oder Verdünnungsmittel geeignet.

Besonders bevorzugtes Lösungs- oder Verdünnungsmittel, insbesondere Lösungsmittel, ist Wasser.

Als Verdünnungsmittel bei der Durchführung der Umsetzung in der Gasphase sind auch Inertgase wie Stickstoff (z.B. über die Sättigung des Reaktorzulaufs hinaus) oder Argon geeignet. Bevorzugt wird die Umsetzung in der Gasphase in Gegenwart von Ammoniak durchgeführt.

Die TEDA-Selektivität, die mit dem erfindungsgemäßen Verfahren erreicht werden kann, liegt bei Werten bis zu > 90 %.

Die Eduktkomponenten oder der Reaktorzulauf werden vorteilhafterweise vortemperiert.

Für die Durchführung des erfindungsgemäßen Verfahrens haben sich weiterhin folgende Reaktionsbedingungen als günstig erwiesen:
- eine WHSV (weight hourly space velocity) bezogen auf in die Umsetzung eingesetzte Amine von 0,01 bis 6 h⁻¹, vorzugsweise 0,05 bis 1 h⁻¹, besonders bevorzugt von 0,1 bis 1 h⁻¹.

Als Reaktoren, in denen das erfindungsgemäße Verfahren durchgeführt wird, eignen sich Rührbehälter, insbesondere Rohrreaktoren und Rohrbündelreaktoren.

Der Zeolith-Katalysator ist im Reaktor bevorzugt als Festbett angeordnet.

Die Umsetzung in der Flüssigphase kann z.B. in der Suspensions-, Riesel- oder Sumpffahrweise erfolgen.

Die bevorzugte Umsetzung in der Gasphase kann in einem Katalysator-Wirbelbett oder bevorzugt -Festbett erfolgen.

Im folgenden Absatz wird zusätzlich beispielhaft geschildert, wie das erfindungsgemäße Verfahren durchgeführt werden kann:

Der Reaktorzulauf (Zusammensetzung: wie oben beschrieben) wird in einem Verdampfer, der gegebenenfalls Bestandteil des eigentlichen Reaktors sein kann, bei einer Temperatur von 250 - 500°C in die Gasphase überführt und auf den Katalysator geleitet. Der am Reaktorausgang gasförmig anfallende Reaktionsaustrag wird durch im Kreislauf gepumpten verflüssigten Reaktionsaustrag bei Temperaturen von 20 - 100°C, bevorzugt bei 80°C gequencht. Dieser verflüssigte Reaktionsaustrag wird wie folgt aufgearbeitet: In einer ersten Destillationsstufe werden Leichtsieder wie Acetaldehyd, Ethylamin, Ammoniak und Wasser sowie heterocyclische Verbindungen, die als Nebenkomponenten in der Synthese gebildet werden, abgetrennt. In einer zweiten Destillationsstufe wird der Reaktionsaustrag von Piperazin befreit, welches erneut dem Reaktorzulauf zugeführt wird. Der Strom des abgetrennten Piperazins kann dabei bis zu 20 Gew.-% TEDA enthalten. (Alternativ ist auch die gleichzeitige Abtrennung von Wasser und Piperazin möglich, die gemeinsam in den Reaktorzulauf zurückgeführt werden können). In einer dritten Destillationsstufe wird das Wertprodukt TEDA destillativ aus dem Reaktionsaustrag gewonnen und bei Bedarf, z.B. in einer nachfolgenden Kristallisationsstufe (z.B. wie weiter unten beschrieben), weiter aufgearbeitet.

Mit dem erfindungsgemäßen Verfahren werden unter anderem folgende Vorteile erzielt:
- nur ein einziges Edukt (PIP) wird eingesetzt
- geringer Wasserüberschuß im Feed (< 60 Gew.-%)
- hoher PIP-Umsatz (> 80 %)
- hohe TEDA-Selektivität (> 90 %)
- einfachere Aufarbeitung des TEDA, da wenig EtPIP gebildet wird
- hohe Katalysator-Standzeit (> 1000 h), Katalysator regenerierbar.

Das erfindungsgemäße Verfahren kann auch mit einem substituierten Piperazin, beispielsweise Aminoethylpiperazin, Bis(Aminoethyl)piperazin, Hydroxyethylpiperazin oder Bis(Hydroxyethyl)piperazin, durchgeführt werden. Dabei entfällt allerdings der erfindungsgemäße Vorteil, dass die Synthese der substituierten Piperazine unnötig wird.

Da durch den Einsatz der vorstehend beschriebenen Katalysatoren die Bildung des unerwünschten Nebenprodukts EtPIP nur in untergeordnetem Maß eintritt, kann die TEDA-Synthese aus PIP unter Bedingungen durchgeführt werden, die einen höheren Umsatz ergeben. Insbesondere lässt sich der Umsatz durch höhere Reaktionstemperaturen steigern. Aufgrund des höheren Umsatzes liegen geringere Kreisströme an PIP vor, weiterhin wird der Reinigungsaufwand für das Endprodukt TEDA vermindert. Der mit dem erfindungsgemäßen Verfahren erzielbare Umsatz als PIP liegt bei Werten über 80 %, vorzugsweise 60 bis 75 %.

Die Erfindung wird nun in den nachstehenden Beispielen näher erläutert.

### Beispiele

### A Katalysatorherstellung

Das Zeolith-Pulver (Fa. ALSIPENTA, Alumosilikat des Typs Na-ZSM-5, Modul 1000) wurde dreimal mit einer 20 %-igen NH₄Cl-Lösung ausgetauscht, gewaschen und bei 500°C für 5 h kalziniert. Anschließend wurde das Pulver dreimal für 8 h einer Säurebehandlung (5 %-ige HCl, Raumtemperatur) unterzogen, jeweils mit Wasser neutral gewaschen und bei 500°C für 3 h kalziniert. Abschließend wurde das Zeolith-Pulver mit 20 Gew.-% SiO₂ (bezogen auf die Gesamtmasse der fertigen 2 mm-Stränge) verstrangt und bei 500°C für 5 h kalziniert.

### B Durchführung des Verfahrens

Die Katalysatoren wurden in einer Gasphasenapparatur eingesetzt (beheizter Rohrreaktor; Länge: 1000 mm, Durchmesser 6 mm). Eduktgemisch: 50 % PIP, 50,0 % Wasser (alle Angaben in Gew.-%). Das wässrige Eduktgemisch wurde direkt in den Reaktor gepumpt und im oberen Teil bei einer Reaktionstemperatur von 350°C bzw. 370°C verdampft, bevor es drucklos auf den Katalysator geleitet wurde. Belastung: 0,2 kg Eduktgemisch/kg Katalysator*h. Die Reaktionsprodukte wurden am Reaktorausgang in einem Kühler kondensiert, gesammelt und ein aliquoter Anteil gaschromatographisch analysiert.

### GC-Analytik:

Säule: RTX-5, 30 m; Temperaturprogramm: 80°C - 5°C/min - 280°C, Detektor: FID.

**Tabelle 1:**

| Synthese von Triethylendiamin (TEDA) aus Piperazin (PIP) | | | | | | | |
|---|---|---|---|---|---|---|---|
| **Beispiel** | **Katalysator** | **Modul** [SiO2/Al2O3] | **WHSV** [h⁻¹] | **Temp.** [°C] | U_{PIP} [%] | S_{TEDA} [%] | S Et-PIP [%] |
| 1 | A (SKO 325) | 1000 | 0.1 | **370** | 71 | 88 | **0,3** |
| 2 | A (SKO 325) | 1000 | 0.1 | **350** | 60 | 88 | **0,2** |
| 3*) | H-ZSM 5 | 90 | ~0,5 | **340** | 43 | 91 | **0,80** |
| 4*) | H-ZSM 5 | 31 | ~0,5 | **310** | 18 | 92 | **0,21** |
| 5*) | H-ZSM 5 | 31 | ~0,5 | **340** | 66 | 88 | **0,91** |

| | | | | | | | |
|---|---|---|---|---|---|---|---|
| *) Vergleichsbeispiel: EP 312734, Tabelle Seite 6 Eintrag Nr. 5, 3 und 6; 3 bar PIP : H₂O = 40:60 Gew.-% | | | | | | | |

## Patentansprüche

1. Verfahren zur Herstellung von Triethylendiamin durch Umsetzung von Piperazin an einem Zeolithkatalysator, der neben SiO₂ ein Oxid mindestens eines weiteren zwei, drei- oder vier-wertigen Metalls M enthält, **dadurch gekennzeichnet, dass** der Zeolith ein molares Verhältnis Si/M von > 100 aufweist.

2. Verfahren nach Anspruch 1, **dadurch gekennzeichnet, dass** M ausgewählt ist aus der Gruppe bestehend aus Al, B, Fe, Co, Ni, V, Mo, Mn, As, Sb, Bi, La, Ga, In, Y, Sc, Cr, Zn, Sn, Be, Ti, Zr, Ge, Hf.

3. Verfahren nach Anspruch 2, **dadurch gekennzeichnet, dass** M ausgewählt ist aus der Gruppe bestehend aus B, Al, Fe, Ga, Ti, Ge, insbesondere Al ist.

4. Verfahren nach einem der Ansprüche 1 bis 3, **dadurch gekennzeichnet, dass** der Zeolith-Katalysator ein molares Verhältnis Si/M von > 200, vorzugsweise 300 bis 40000, insbesondere 400 bis 5000, aufweist.

5. Verfahren nach einem der Ansprüche 1 bis 4, **dadurch gekennzeichnet, dass** ein Zeolith des Pentasil-Typs, insbesondere des Typs ZSM-5 oder ZSM-11 oder eine Mischstruktur davon, eingesetzt wird.

6. Verfahren nach einem der Ansprüche 1 bis 5, **dadurch gekennzeichnet, dass** der Katalysator mindestens teilweise in der H-Form eingesetzt wird.

7. Verfahren nach einem der Ansprüche 1 bis 6, **dadurch gekennzeichnet, dass** dieses bei Temperaturen von 250 bis 500°C, vorzugsweise 300 bis 450 °C, insbesondere 330 bis 400°C, und bei Drücken von 0,01 bis 50 bar, vorzugsweise 0,5 bis 20 bar, insbesondere bei Atmosphärendruck zuzüglich den Druckverlust der über das Katalysatorbett entsteht, durchgeführt wird.

8. Verfahren nach einem der Ansprüche 1 bis 7, **dadurch gekennzeichnet, dass** Piperazin im Gemisch mit Wasser und/oder einem organischen Verdünnungsmittel eingesetzt wird, vorzugsweise als Gemisch mit mindestens 10 Gew.-% Wasser, mehr bevorzugt 20 bis 60 Gew.-% Wasser, insbesondere 30 bis 50 Gew.-% Wasser.

9. Verfahren nach einem der Ansprüche 1 bis 8, **dadurch gekennzeichnet, dass** der Umsatz an Piperazin bei Werten über 80 %, vorzugsweise 60 bis 80 %, liegt.

10. Verfahren nach einem der Ansprüche 1 bis 9, **dadurch gekennzeichnet, dass** das Verfahren diskontinuierlich oder kontinuierlich, vorzugsweise kontinuierlich, mehr bevorzugt in der Gasphase, insbesondere an einem Katalysator-Wirbelbett oder -Festbett, durchgeführt wird.

## Claims

1. A process for the preparation of triethylenediamine by reacting piperazine over a zeolite catalyst which, in addition to SiO₂, comprises an oxide of at least one further divalent, trivalent or tetravalent metal M, wherein the zeolite has a molar Si/M ratio of > 100.

2. The process according to claim 1, wherein M is selected from the group consisting of Al, B, Fe, Co, Ni, V, Mo, Mn, As, Sb, Bi, La, Ga, In, Y, Sc, Cr, Zn, Sn, Be, Ti, Zr, Ge and Hf.

3. The process according to claim 2, wherein M is selected from the group consisting of B, Al, Fe, Ga, Ti and Ge, and in particular is Al.

4. The process according to any of claims 1 to 3, wherein the zeolite catalyst has a molar Si/M ratio of > 200, preferably from 300 to 40 000, in particular from 400 to 5 000.

5. The process according to any of claims 1 to 4, wherein a zeolite of the Pentasil type, in particular of the ZSM-5 or ZSM-11 type or a mixed structure thereof, is used.

6. The process according to any of claims 1 to 5, wherein the catalyst is used at least partly in the H form.

7. The process according to any of claims 1 to 6, which is carried out at from 250 to 500°C, preferably from 300 to 450°C, in particular from 400°C, and at from 0.01 to 50, preferably from 0.5 to 20, bar, in particular at atmospheric pressure, plus the resulting pressure drop across the catalyst bed.

8. The process according to any of claims 1 to 7, wherein piperazine is used as a mixture with water and/or an organic diluent, preferably as a mixture with at least 10, preferably from 20 to 60, in particular from 30 to 50, % by weight of water.

9. The process according to any of claims 1 to 8, wherein the piperazine conversion is more than 80%, preferably from 60 to 80%.

10. The process according to any of claims 1 to 9, which is carried out batchwise or continuously, preferably continuously, more preferably in the gas phase, in particular over a fluidized catalyst bed or a fixed catalyst bed.

## Revendications

1. Procédé de fabrication de triéthylènediamine par réaction de pipérazine sur un catalyseur de zéolite, qui contient, outre du SiO₂ un oxyde d'au moins un autre métal M bivalent, trivalent ou quadrivalent, **caractérisé en ce que** la zéolite présente un rapport molaire Si/M > 100.

2. Procédé selon la revendication 1, **caractérisé en ce que** M est choisi dans le groupe constitué de Al, B, Fe, Co, Ni, V, Mo, Mn, As, Sb, Bi, La, Ga, In, Y, Sc, Cr, Zn, Sn, Be, Ti, Zr, Ge, Hf.

3. Procédé selon la revendication 2, **caractérisé en ce que** M est choisi dans le groupe constitué de B, Al, Fe, Ga, Ti, Ge, en particulier Al.

4. Procédé selon l'une quelconque des revendications 1 à 3, **caractérisé en ce que** le catalyseur de zéolite présente un rapport molaire Si/M supérieur à 200, de préférence de 300 à 40 000, en particulier de 400 à 5000.

5. Procédé selon l'une quelconque des revendications 1 à 4, **caractérisé en ce que** l'on utilise une zéolite de type pentasil, en particulier de type ZSM-5 ou ZSM-11 ou une de leurs structures mixtes.

6. Procédé selon l'une quelconque des revendications 1 à 5, **caractérisé en ce que** l'on utilise le catalyseur au moins partiellement sous la forme H.

7. Procédé selon l'une quelconque des revendications 1 à 6, **caractérisé en ce qu'**il est effectué à des températures de 250 à 500°C, de préférence de 300 à 450°C, en particulier de 330 à 400°C, et à des pressions de 0,01 à 50 bars, de préférence de 0,5 à 20 bars, en particulier à pression atmosphérique en plus de la perte de pression qui apparaît sur le lit de catalyseur.

8. Procédé selon l'une quelconque des revendications 1 à 7, **caractérisé en ce que** l'on utilise de la pipérazine en mélange avec de l'eau et/ou avec un diluant organique, de préférence en mélange avec au moins 10% en poids d'eau, mieux encore 20 à 60% en poids d'eau, en particulier 30 à 50% en poids d'eau.

9. Procédé selon l'une quelconque des revendications 1 à 8, **caractérisé en ce que** la conversion de la pipérazine se situe à des valeurs supérieures à 80%, de préférence de 60 à 80%.

10. Procédé selon l'une quelconque des revendications 1 à 9, **caractérisé en ce que** l'on effectue le procédé en discontinu ou en continu, de préférence en continu, mieux encore en phase gazeuse, en particulier sur un lit fluidisé ou un lit fixe de catalyseur.
